Europäisches Patentamt

European Patent Office

Office européen des brevets

(19)

(11) **EP 1 393 769 A1**

(12) **EUROPEAN PATENT APPLICATION**
published in accordance with Art. 158(3) EPC

(43) Date of publication:
**03.03.2004 Bulletin 2004/10**

(51) Int Cl.⁷: **A61M 25/00**

(86) International application number:
**PCT/JP2002/005477**

(21) Application number: **02730895.6**

(22) Date of filing: **04.06.2002**

(87) International publication number:
**WO 2002/098498 (12.12.2002 Gazette 2002/50)**

(84) Designated Contracting States:
**AT BE CH CY DE DK ES FI FR GB GR IE IT LI LU MC NL PT SE TR**

(30) Priority: **04.06.2001 JP 2001168823**

(71) Applicant: **Japan Lifeline Co., Ltd
Toshima-ku, Tokyo 171-0014 (JP)**

(72) Inventors:
• **KAWABATA, Takashi
Hasuda-shi, Saitama 349-0104 (JP)**
• **SHIRAKI, Kaneto
Nerima-ku, Tokyo 178-0061 (JP)**
• **MAKINO, Morihide
Kita-ku, Tokyo 115-0055 (JP)**

(74) Representative: **Olgemöller, Luitgard, Dr.
Leonhard - Olgemöller - Fricke,
Patentanwälte,
Postfach 10 09 62
80083 München (DE)**

(54) **BALLOON CATHETER AND PROCESS FOR PRODUCING BALLOON FOR BALOON CATHETERS**

(57)    The present invention provides a balloon catheter in which the balloon is a thin film and can be compactly folded up when the balloon is deflated, while the thin film has high strength so that the balloon has excellent pressure resistance when inflated, and which is safe and easy to manipulate, and also provides a method for manufacturing a balloon for use in the balloon catheter.

A method for manufacturing a balloon for use in a balloon catheter, wherein a thin film of a high-strength resin is formed by deposition polymerization or coating over a balloon mold formed from a water-soluble polymer resin, after which the balloon mold is removed.

Figure 1

**Description**

TECHNICAL FIELD

This invention relates to a balloon catheter and a method for manufacturing a balloon used in a balloon catheter, and more particularly to a balloon catheter having high strength despite being a thin film, which can be used in percutaneous transluminal coronary angioplasty (PTCA), which is a treatment for heart disease and other such afflictions, and to a method for manufacturing a balloon used in the balloon catheter.

BACKGROUND ART

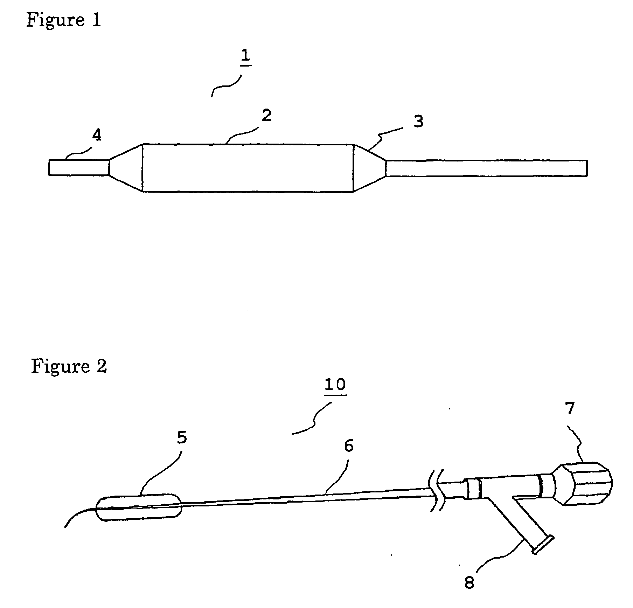

Balloon catheters are used primarily in the angioplasty treatment of constricted or blocked blood vessels. A typical balloon catheter is shown in Fig. 2. A balloon catheter 10 has a balloon component 5 near the distal end of a catheter component 6, and a rotating adapter 7 and a female luer adapter 8 are provided near the proximal end of the catheter component 6 for connecting tubes. The balloon component 5 shown in Fig. 2 is in a state in which the balloon has been inflated, although ordinarily the balloon is folded up. During treatment, the balloon component 5 of the balloon catheter 10 is inserted into the constricted portion of a blood vessel via one of the patient's arteries. The balloon component 5 is inflated by injecting a balloon inflation liquid or other such pressure fluid into the balloon component 5, which spreads open the constricted or blocked portion of the blood vessel.

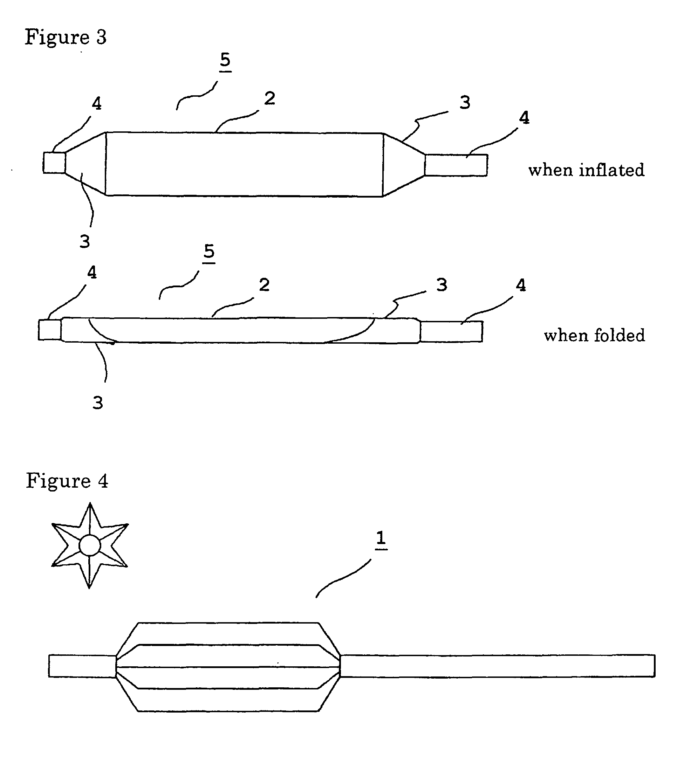

Fig. 3 illustrates the typical shape of a balloon. The balloon component 5 is hollow and consists of a large diameter section 2, small diameter sections 4, and tapering sections 3 between the large diameter section 2 and the small diameter sections 4. The catheter component 6 is connected to the small diameter section 4. A piece of tubing used for transporting a guide wire or a balloon inflation liquid is inserted into the hollow space. The large diameter section 2 and the tapering sections 3 fold up as shown in Fig. 3. The balloon is made of a thermoplastic resin with excellent moldability, such as polyethylene, polyethylene terephthalate, or a polyolefin copolymer. The most common method for molding the balloon is blow molding, in which the thermoplastic resin is molded into a tubular body by extrusion molding, and is subjected to electron beam crosslinking if needed, after which the portion that will become the large diameter section expanded by being heated and pressed. If the thermoplastic resin is not subjected to electron beam crosslinking, it is possible to employ a method in which the above-mentioned tubular body is placed in a specific mold, and the portion corresponding to the large diameter section is heated and pressed, which expands the portion corresponding to the large diameter section and forms this large diameter section.

The primary use of balloon catheters is to insert them into body passages where treatment is needed, such as constricted blood vessels. The treatment is performed by expanding the balloon component by injecting a pressure fluid into the balloon component that has been inserted and located at the treatment site, such as a constriction in a blood vessel. Therefore, one of the mechanical properties necessary to the balloon is that it be strong enough not to burst when pressure is applied. If the balloon walls are made thicker, the balloon can be made strong enough not to burst when the pressure fluid is injected. However, if we consider the operability of the balloon catheter, its effect on the body, etc., it is preferable if the balloon can be folded up so that its large diameter section is as small as possible when inserted into the body, and if it can be refolded as compactly after being inflated as before inflation. Making the walls of the balloon thinner is preferable in order to facilitate folding the balloon more compactly.

In the past, however, the thickness of a balloon could only be reduced to about 30 μm, even with materials having good workability such as polyethylene. Furthermore, a drawback to reducing the thickness to this level was that it decreased the strength and made the balloon more prone to bursting during treatment. On the other hand, if a material with high strength was used in an effort to boost the strength of the balloon, a balloon with thin walls could not be produced because the workability of high-strength materials was poor with conventional blow molding methods.

Therefore, it is an objective of the present invention to provide a balloon catheter in which the balloon is a thin film and which can be compactly folded up when the balloon is deflated, while the thin film has high strength so that the balloon has excellent pressure resistance when inflated, and which is safe and easy to manipulate, and also provides a method for manufacturing a balloon for use in the balloon catheter.

DISCLOSURE OF THE INVENTION

The present invention provides a method of manufacturing a balloon for use in a balloon catheter, wherein a thin film of a high-strength resin is molded over the surface of a balloon mold formed from a polymer resin, after which the balloon mold is removed.

The present invention also provides a balloon catheter which makes use of a balloon manufactured by the above-mentioned manufacturing method.

BRIEF DESCRIPTION OF THE DRAWINGS

Fig. 1 is an overall structural diagram of the balloon mold formed from a polymer resin pertaining to the present invention.
Fig. 2 is an overall structural diagram of a conventional balloon catheter.
Fig. 3 is a diagram illustrating the state of a conventional balloon when inflated and when folded.
Fig. 4 is an overall structural diagram of a variation example of the balloon. mold formed from a polymer resin pertaining to the present invention.

BEST MODE FOR CARRYING OUT THE INVENTION

The present invention provides a method for manufacturing a balloon for use in a balloon catheter, wherein a thin film of a high-strength resin is molded over the outer surface of a balloon mold formed of a polymer resin, after which the balloon mold is removed, and a balloon catheter which makes use of a balloon manufactured by this manufacturing method.

The above-mentioned polymer resin can be any water-soluble polymer or polymer that has a glass transition point higher than the temperature at which the thin film of the above-mentioned high-strength resin is formed, and that dissolves, melts, or decomposes at a melting point lower than the melting point of the high-strength resin. If the balloon mold is formed of a water-soluble polymer, for example, then just the balloon mold on which the thin film of high-strength resin has been formed by deposition polymerization or the like can be eluted in water and removed from the balloon merely by immersing the balloon mold in water and thereby bringing the balloon mold into contact with water. Also, if the balloon mold is formed of a low-temperature-melting polymer whose glass transition point is higher than the deposition polymerization temperature and which melts at a melting point lower than the melting point of the high-strength resin, then just the balloon mold on which the thin film of high-strength resin has been formed by deposition polymerization can be melted and removed from the balloon by heating this balloon mold to a temperature that is over the above-mentioned melting point but lower than the glass transition point of the high-strength resin. This low-temperature-melting polymer can also be a water-soluble polymer as well, depending on the type thereof.

There are no particular restrictions on the type of the above-mentioned water-soluble polymer as long as it allows a balloon mold to be formed, but examples include carboxymethyl cellulose and other cellulose derivatives, polyvinylpyrrolidone, polyvinyl alcohol, polymethacrylic acid, sodium polyethylenesulfonate, polyethylene oxide, polyvinyl acetate, and copolymers of polyvinyl alcohol and polyethylene oxide. One or more types can be selected from this group. Of these, a copolymer of polyvinyl alcohol and polyethylene oxide is preferred in terms of its moldability and water solubility.

The above-mentioned water-soluble polymer preferably has an apparent melt viscosity of 5000 to 40,000 centipoise/100 seconds.

There are no particular restrictions on the above-mentioned low-temperature-melting polymer as long as it allows a balloon mold to be formed and can be melted and removed after vapor deposition without any deformation occurring during deposition polymerization, but examples include low molecular weight polyethylene and waxes.

The balloon mold may have the same shape as that of the inflated balloon. shown in Fig. 1, or may have the shape of the deflated balloon shown in Fig. 4. When a balloon is formed on a balloon mold having the shape of a deflated balloon, there is no need to subsequently provide fold lines for evenly folding up the balloon, which shortens the balloon catheter manufacturing process. The balloon mold may be either solid or hollow, but a hollow mold is preferable because it makes easier to remove the polymerized balloon mold by elution or melting.

The balloon mold can be formed by subjecting the above-mentioned polymer resin to injection molding or extrusion molding. If the polymer resin has a high melt viscosity, injection molding is better, while extrusion molding is more suited to the resin of a low melt viscosity. This injection molding is carried out by melting the molding material in a heated cylinder to convert it into a fluid, plastic state, injecting the material into a metal mold of the desired shape, and then cooling and solidifying the material in the mold. When a water-soluble polymer is used for the molding material, it is pre-dried prior to being melted. The water-soluble polymer that has absorbed moisture is kept from foaming during injection molding by this pre-drying. The injection conditions will vary with the type of polymer resin, but an example condition is pre-drying for at least 3 hours at a temperature of 120 to 150°C, melting at a temperature of 220 to 240°C, injecting at a pressure of 50 to 200 MPa, and cooling for at least 5 seconds.

Deposition polymerization is a method for forming a polymer by evaporating a raw material monomer or oligomer in a vacuum of $133.322 \times 10^{-2}$ to $133.322 \times 10^{-6}$ Pa and carrying out a polymerization reaction on the intended surface. To conduct deposition polymerization makes it possible to produce a balloon with a high-strength resin that could not be used in the past because of poor workability. Deposition polymerization ensures good coverage, which means that even when a balloon mold having a complex shape such as that in Fig. 4 is used, the material will be uniformly supplied even to tight spaces, so it is possible to form a uniform thin film.

There are no particular restrictions on the high-strength resin formed by deposition polymerization, but examples include polyimides, polyamides, aromatic polyamides, polyureas, polyparaxylenes, and derivatives thereof. These high-strength resins are characterized in that their tensile strength is at least 100 MPa. Of these, aromatic polyamides and polyparaxylenes are preferable because of their high strength.

There are no particular restrictions on the monomer and oligomer supplied to the deposition polymerization to form a film of the high-strength resin, as long as it is a monomer or oligomer with which the high-strength resin, or deposition polymer, can be formed efficiently. Examples of monomers that can be used in the above-mentioned deposition polymerization include combinations of an aromatic tetracarboxylic dianhydride, such as pyromellitic anhydride, and an aromatic diamine, such as 4,4'-diaminodiphenyl ether, to obtain a polyamide acid which produces a polyimide after being heated and dehydrated, while examples of those that can be used to obtain a polyurea such as an aromatic polyurea include combinations of an aromatic diamine, such as 4,4'-diaminodiphenylmethane, and an aromatic diisocyanate, such as 4,4'-diphenylmethane diisocyanate, and examples of those that can be used to obtain an aromatic polyamide include combinations of an aromatic diamine, such as paraphenylenediamine, and an aromatic dicarboxylic dichloride, such as dichloride terephthalate.

A polyparaxylene is a favorable example for a thin film of a high-strength resin. This polyparaxylene polymer can be obtained from one of the paraxylene dimers expressed by Formulas 1 to 5 below, for example. Me is a methyl group in Formula 4.

(1)

(2)

(3)

$$CH_2 - \text{(ring, } Me\text{)} - CH_2$$
$$CH_2 - \text{(ring, } Me\text{)} - CH_2$$

(4)

$$CF_2 - \text{(ring)} - CF_2$$
$$CF_2 - \text{(ring)} - CF_2$$

(5)

Any monomer or oligomer having a functional group that allows the formation of an amide bond, imide bond, or urea bond can be used in the deposition polymerization of the present invention. For instance, examples of monomers or oligomers having the same functional groups within a given molecule include diamine derivatives, such as aliphatic diamines, alicyclic diamines, aromatic diamines and heterocyclic diamines, dicarboxylic acid derivatives such as aliphatic dicarboxylic acids, alicyclic dicarboxylic acids, aromatic dicarboxylic acids, heterocyclic dicarboxylic acids and carboxylic esters of these, and diisocyanate derivatives such as aliphatic diisocyanates, alicyclic diisocyanates, aromatic diisocyanates and heterocyclic diisocyanates. Examples of monomers or oligomers having different functional groups within a given molecule include carboxylic acid isocyanates, carboxylic acid amines and hydroxy-carboxylic acids.

The vapor deposition conditions will vary with the type of monomer, but the deposition temperature at least should be lower than the glass transition point of the polymer used to form the balloon mold, so that there will be no deformation of the balloon mold. The deposited film thickness will also vary with the type of high-strength resin, but a range of 10 to 50 μm is favorable. Pressure resistance will be lacking if the thickness is less than 10 μm, but if 50 μm is exceeded, the folded balloon will be bulky and folding will be more difficult.

The deposited film is not limited to a single-layer film of high-strength resin, and a multilayer film can be produced, if necessary. For instance, a film with good biocompatibility, such as polyimide fluoride, can be deposited over the high-strength resin that has been deposited on the balloon mold. Forming a film with good biocompatibility on the balloon surface is advantageous in that it improves the compatibility of the balloon catheter with biological tissue, and also provides lower friction, so there is less burden imposed on the body.

After the thin film of a balloon has been formed by deposition polymerization, the balloon mold is removed from the balloon. If the balloon mold has been formed from a water-soluble polymer, it is removed by being dissolved in an aqueous solution. The removal of a water-soluble polymer is accomplished by soaking in distilled water at a temperature of 25 to 50°C for 1 to 24 hours. If the balloon mold has been formed from a low-temperature-melting polymer, then the balloon mold on which the balloon thin film has been formed is heated to a temperature that is higher than the melting point of the low-temperature-melting polymer but lower than the glass transition point of the high-strength resin, which melts and removes the low-temperature-melting polymer.

Furthermore, the thin film of a high-strength resin may be formed by coating the surface of the balloon mold on which the polymer resin has been formed. An aromatic polyamide can be used favorably as a coating material in this case. A balloon produced in this way can be worked into a balloon catheter by a known method.

Examples

Example 1 - Formation of balloon by deposition polymerization

(1-1) Producing the balloon mold

A polyvinyl alcohol-polyethylene oxide copolymer that had been pre-dried for 3 hours at 150°C was subjected to injection molding under conditions comprising a melting temperature of 220°C, an injection pressure of 100 MPa, and a cooling time of 5 seconds, which produced the balloon mold shown in Fig. 1, having a small-diameter section with a diameter of 0.8 mm and a large diameter section with a diameter of 3.0 mm.

(1-2) Deposition polymerization of the high-strength resin

Paraphenylenediamine and dichloride terephthalate were used for the raw material monomers. An aromatic polyamide was deposition-polymerized in a thickness of 20 μm on the balloon mold for 1 hour at a deposition temperature of normal temperature under a vacuum pressure of $133.322 \times 10^{-5}$ Pa. A heat treatment was conducted for 1 hour at 150°C upon completion of the deposition in order to remove the HCl generated during deposition.

(1-3) Removal of the balloon mold

The balloon mold with a surface on which the film had been deposited was soaked for 24 hours in 50°C distilled water, which dissolved away the balloon mold composed of the polyvinyl alcohol-polyethylene oxide copolymer, forming a balloon having a wall thickness of 20 μm.

Example 2 - Formation of balloon using Parylene

(2-1) Producing the balloon mold

The steps of producing the balloon mold were the same as those in Example 1.

(2-2) Deposition polymerization of Parylene

Deposition polymerization of the Parylene C represented by formula 2 was performed for 4 hours on the outer surface of this balloon mold, and then a cover film having a thickness of 20 μm was formed. The pressure resistance of this balloon was 25 kg/cm$^2$. The deposition polymerization apparatus was equipped with an evaporator, a pyrolyzing furnace, a vapor deposition chamber, a cooling trap, and a vacuum pump. The evaporation with the evaporator was carried out at 200°C under 133.322 Pa, the pyrolysis in the pyrolyzing furnace at 680°C under 66.661 Pa, and the vapor deposition in the deposition chamber at 25°C under 13.3322 Pa.

(2-3) Removal of the balloon mold

The steps for the removal were the same as those in Example 1.

Example 3 - Formation of balloon by coating

(3-1) Producing the balloon mold

The steps were the same as those in Example 1.

(3-2) Producing a coating solution

m-Phenylenediamine (MPD) and isophthalic acid chloride (IPC) were polymerized in dimethylacetamide solvent, and was obtained the aromatic polyamide represented by formula 6. The above-mentioned solvent was added to the obtained, and the viscosity was adjusted to 1000 cps. Thus a coating solution was produced.

(3-3) Formation of the balloon

The above-mentioned balloon mold was immersed in the above-mentioned coating solution and coated with it, whereby a dry film having a thickness of approximately 20 μm was produced. The solvent was removed at 80°C initially, after which the rest was removed under a reduced pressure.

(3-4) Removal of the balloon mold

The steps were the same as those in Example 1.

(4) Balloon pressure test

The medical balloons obtained in Examples 1 to 3 above were subjected to the following pressure test with a pressure tester (Hydraulic Burst-Leak Tester, Model 1000) made by Crescent Design.
In this pressure test, one end of the balloon was sealed off, 37°C physiological saline was introduced from the other end at a water pressure of 10 kg/cm$^2$, the water pressure was raised by 1 kg/cm$^2$ at 30-second intervals, and the balloon was checked for bursting. The same test was performed on 10 samples.
None of the 10 sample balloons in each of Examples 1 to 3 burst at a water pressure of 15 kg/cm$^2$.
For the purpose of comparison, a commercially available balloon with a wall thickness of 30 μm produced by a conventional blow molding method was subjected to the same pressure test, whereupon 5 of the 10 samples burst before the water pressure reached 15 kg/cm$^2$. Despite having a film thickness of only two-thirds of that of the conventional product, the balloons of the present invention still exhibited higher pressure-resistance.

INDUSTRIAL APPLICABILITY

A balloon manufactured by the balloon manufacturing method of the present invention is thin yet strong, so that it is possible to obtain a balloon catheter in which the large diameter section of the balloon can be folded compactly, and even after it has been inflated, it can be refolded just as compactly as before inflation, and which affords excellent safety, with no danger of bursting during use, as well as being easier to manipulate than a conventional product.

**Claims**

1. A method for manufacturing a balloon for use in a balloon catheter, comprising molding a thin film of a high-strength resin over the surface of a balloon mold formed from a polymer resin, and removing the balloon mold.

2. The method for manufacturing a balloon for use in a balloon catheter according to Claim 1, wherein the polymer resin is a water-soluble polymer.

3. The method for manufacturing a balloon for use in a balloon catheter according to Claim 2, wherein the water-soluble polymer is at least one selected from the group consisting of a cellulose derivative such as carboxymethyl cellulose, a polyvinylpyrrolidone, a polyvinyl alcohol, a polymethacrylic acid, a sodium polyethylenesulfonate, a polyethylene oxide, a polyvinyl acetate, and a copolymer of polyvinyl alcohol and polyethylene oxide.

4. The method for manufacturing a balloon for use in a balloon catheter according to Claim 2 or 3, wherein the water-soluble polymer has an apparent melt viscosity of 5000 to 40,000 centipoise/100 seconds.

5. The method for manufacturing a balloon for use in a balloon catheter according to any one of Claims 1 to 4, wherein the polymer resin is a polymer with a low melting point.

**6.** The method for manufacturing a balloon for use in a balloon catheter according to Claim 5, wherein the polymer with a low melting point is a low molecular weight polyethylene or wax.

**7.** The method for manufacturing a balloon for use in a balloon catheter according to any one of Claims 1 to 6, wherein the high-strength resin is molded by deposition polymerization or coating.

**8.** The method for manufacturing a balloon for use in a balloon catheter according to Claim 1, wherein the polymer resin has a glass transition point higher than a temperature at which the thin film of the high-strength resin is molded, and the polymer resin dissolves, melts, or decomposes at a melting point lower than the glass transition point of the high-strength resin.

**9.** The method for manufacturing a balloon for use in a balloon catheter according to any one of Claims 1 to 8, wherein the high-strength resin is one selected from the group consisting of a polyimide, a polyamide, an aromatic polyamide, a polyurea, and a polyparaxylylene derivative.

**10.** The method for manufacturing a balloon for use in a balloon catheter according to any one of Claims 1 to 9, wherein deposition polymerization is performed over a balloon mold having a shape which the balloon can take when the balloon is inflated.

**11.** The method for manufacturing a balloon for use in a balloon catheter according to any of Claims 1 to 9, wherein deposition polymerization is performed over a balloon mold having a shape which the balloon can take when the balloon is deflated.

**12.** The method for manufacturing a balloon for use in a balloon catheter according to any of Claims 1 to 11, wherein the thin film of the high-strength resin is a multi-layer film.

**13.** The method for manufacturing a balloon for use in a balloon catheter according to any of Claims 1 to 12, further comprising forming a film with good biocompatibility over the thin film of the high-strength resin.

**14.** A balloon catheter, which makes use of a balloon manufactured by the manufacturing method according to any one of Claims 1 to 13.

Figure 1

Figure 2

Figure 3

when inflated

when folded

Figure 4

## INTERNATIONAL SEARCH REPORT

| International application No. |
|---|
| PCT/JP02/05477 |

| A. CLASSIFICATION OF SUBJECT MATTER |
|---|
| Int.Cl$^7$ A61M25/00 |

According to International Patent Classification (IPC) or to both national classification and IPC

| B. FIELDS SEARCHED |
|---|

Minimum documentation searched (classification system followed by classification symbols)
Int.Cl$^7$ A61M25/00

Documentation searched other than minimum documentation to the extent that such documents are included in the fields searched

| Jitsuyo Shinan Koho | 1922–1996 | Toroku Jitsuyo Shinan Koho | 1994–2002 |
|---|---|---|---|
| Kokai Jitsuyo Shinan Koho | 1971–2002 | Jitsuyo Shinan Toroku Koho | 1996–2002 |

Electronic data base consulted during the international search (name of data base and, where practicable, search terms used)

| C. DOCUMENTS CONSIDERED TO BE RELEVANT |
|---|

| Category* | Citation of document, with indication, where appropriate, of the relevant passages | Relevant to claim No. |
|---|---|---|
| X | JP 11-319102 A (Toray Industries, Inc.), 24 November, 1999 (24.11.99), Full text; Figs. 1 to 9 | 1,5-8,12,14 |
| Y | Full text; Figs. 1 to 9 (Family: none) | 2-4,9-11,13 |
| Y | WO 95/07170 A1 (Minnesota Mining and Manufacturing Co.), 16 March, 1995 (16.03.95), Full text & JP 7-80598 A | 2-4 |
| Y | EP 768097 A2 (Terumo Kabushiki Kaisha), 16 April, 1997 (16.04.97), Full text; Fig. 1 & JP 09-164191 A & US 5879369 A1 | 9,13 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 03 September, 2002 (03.09.02) | 01 October, 2002 (01.10.02) |

| Name and mailing address of the ISA/ | Authorized officer |
|---|---|
| Japanese Patent Office | |
| Facsimile No. | Telephone No. |

Form PCT/ISA/210 (second sheet) (July 1998)

## INTERNATIONAL SEARCH REPORT

| International application No. |
| --- |
| PCT/JP02/05477 |

C (Continuation).  DOCUMENTS CONSIDERED TO BE RELEVANT

| Category* | Citation of document, with indication, where appropriate, of the relevant passages | Relevant to claim No. |
| --- | --- | --- |
| Y | JP 7-178174 A (Terumo Kabushiki Kaisha), 18 July, 1995 (18.07.95), Par. No. [0018] (Family: none) | 10 |
| Y | JP 9-122243 A (Nippon Zeon Co., Ltd.), 13 May, 1997 (13.05.97), Full text; Figs. 1 to 8 (Family: none) | 11 |
| A | WO 86/02580 A1 (Polymed Laboratories), 09 May, 1986 (09.05.86), Full text; Figs. 1 to 7 & JP 62-501054 A | 2-4 |
| A | JP 9-31341 A (Bridgestone Corp.), 04 February, 1997 (04.02.97), (Family: none) | 2-4 |

Form PCT/ISA/210 (continuation of second sheet) (July 1998)